# EUROPÄISCHE PATENTANMELDUNG

(11) **EP 3 069 758 A1**
(43) Veröffentlichungstag der Anmeldung: **21.09.2016**
(21) Anmeldenummer: 16157865.3
(22) Anmeldetag: 29.02.2016
(51) Int. Cl.: A61N 1/375, H01R 13/52, H01B 17/26

(54) **GESTANZTE FLANSCHE FÜR ELEKTRISCHE DURCHFÜHRUNGEN MIT INTEGRIERTEM MASSESTIFT**

(30) Priorität: 20.03.2015 US 201562135716 P
(71) Anmelder: BIOTRONIK SE & Co. KG, 12359 Berlin (DE)
(72) Erfinder: Kronmüller, Daniel, 90409 Nürnberg (DE); Arnold, Michael, 91056 Erlangen (DE); Fries, Lilli, 90547 Stein (DE)
(74) Vertreter: Galander, Marcus

(57) **Zusammenfassung**

Durchführung eines implantierbaren medizinelektronischen Geräts, mit einem Isolierkörper, einem den Isolierkörper umfassenden Durchführungs-Flansch und mindestens einem den Isolierkörper durchstoßenden Anschlusselement zum externen Anschluss eines elektrischen oder elektronischen Bauelements des Geräts, wobei der Durchführungs-Flansch mindestens ein vorgestanztes und gebogenes und/oder abgekantetes und/oder tiefgezogenes Blechteil, insbesondere aus einem Titanblech, oder Titan-Legierungs-Blech, aufweist und das Blechteil einen einstückig angeformten und gegenüber der Erstreckungsebene der Durchführung abgewinkelten Fortsatz aufweist, der als Masseanschlussfläche ausgebildet ist.

## Beschreibung

Die Erfindung betrifft eine Durchführung für ein implantierbares elektromedizinisches Gerät. Ein solches Gerät umfasst typischerweise ein Gerätegehäuse, in dem elektronische und elektrische Funktionseinheiten untergebracht sind, einen Gerätekopf mit mindestens einer Elektrode oder einem Leitungsanschluss und eine zwischen Gerätegehäuse und Gerätekopf angeordnete Durchführung für mindestens ein die Elektroden oder den Leitungsanschluss mit einer Funktionseinheit verbindendes elektrisches Leiterelement. Eine derartige Durchführung umfasst einen Keramik- oder Glas-Isolierkörper, einen den Isolierkörper umfassenden Durchführungs-Flansch und mindestens ein den Isolierkörper durchstoßendendes Anschlusselement zum externen Anschluss eines elektrischen oder elektronischen Bauelements des Geräts. Des Weiteren betrifft die Erfindung ein Verfahren zur Herstellung einer solchen Durchführung.

Implantierbare Geräte der oben bezeichneten Art sind insbesondere als Herzschrittmacher oder implantierbare Kardioverter (speziell Defibrillatoren) seit langem in massenhaftem Einsatz. Es kann sich hierbei aber auch um eine weniger komplexe Vorrichtung, wie etwa eine Elektroden- oder Sensorleitung, handeln. Neben dem Einsatz von Durchführungen in Geräten für die Herztherapie finden Durchführungen auch in Cochlearimplantaten Anwendung.

Die meisten praktisch bedeutsamen implantierbaren elektromedizinischen Geräte sind dazu vorgesehen, über geeignet platzierte Elektroden elektrische Impulse an reizbares Körpergewebe abzugeben. Um diese Funktion auszuführen, sind im Gehäuse des Gerätes elektronische/elektrische Funktionseinheiten zur Erzeugung der Impulse und zur geeigneten Steuerung der Impulserzeugung untergebracht, und außen am Gerät sind unmittelbar Elektroden oder Anschlüsse für mindestens eine Elektrodenleitung vorgesehen, in deren distalem Endabschnitt die Elektroden zur Impulsübertragung an das Gewebe angebracht sind. Die elektronischen/elektrischen Funktionseinheiten im Geräteinneren sind in einer Weise mit den äußeren Elektroden oder Elektrodenleitungsanschlüssen zu verbinden, die unter den speziellen Bedingungen des implantierten Zustandes eine absolut und dauerhaft verlässliche Funktion gewährleistet. Die Anschlüsse oder Elektrodenleitungen können auch genutzt werden, um elektrische Impulse und Reize im Körper des Patienten gezielt zu messen und über einen längeren Zeitraum aufzuzeichnen oder auszuwerten, um eine individuell angepasste Therapie zu wählen und den Erfolg der Behandlung zu überprüfen.

Bekannt sind insbesondere Durchführungen, deren Grund- und Isolationskörper im Wesentlichen aus Keramik oder Glas besteht, wobei auch mehrschichtige bzw. mehrteilige Aufbauten unter Einsatz von Metallen oder Metalloxiden entwickelt wurden und eingesetzt werden. Derartige bekannte Durchführungen erfüllen weitgehend die an sie gestellten Anforderungen von Hermetizität, Biokompatibilität, Signalübertragung, und Langzeitstabilität.

Zur Bereitstellung eines Massepotentials für die elektronischen/elektrischen Bauelemente und Baugruppen des Gerätes wird eine Verbindung zu dessen metallischem Gehäuse hergestellt, und zwar typischerweise durch ein spezielles Masse-Verbindungsmittel im Bereich der Durchführung, speziell einen sogenannten Massestift (Massepin). Ein Massestift, beispielsweise aus Niob oder Pt/Ir, wird bei einigen Typen bekannter Geräte mittels Widerstandsschweißen am Gehäuse aus Titan gefügt. Die elektrische Verbindung zwischen Gehäuse und Platine entsteht nach dem Schweißen oder Löten des Massepins auf der Platine und dem Einschweißen des Flansches mit dem Gehäuse. Alternativ erfolgt die Masseanbindung mittels eines Stiftes, welcher während des Bestückens in eine Sacklochbohrung montiert und im Hochtemperaturlötverfahren mit dem Flansch verlötet wird. Die elektrische Verbindung durch das Gehäuse entsteht nach dem Weichlöten der Durchführung auf der Platine und dem Einschweißen des Flansches mit dem Gehäuse.

Zur Herstellung der Masseanbindung auf diese Weise wird eine zusätzliche Komponente benötigt. Diese muss in der Lagerhaltungs-Datenbasis, im Lager, in der Konstruktion usw. geführt werden. Während des Produktionsprozesses kann es zu Verwechselungen mit anderen Komponenten kommen, welche andere Maße haben. Da der Massestift nicht durchgängig ist, handelt es sich im Vergleich zu anderen Stiften um ein kürzeres Bauteil. Da der Massestift nur einmal pro Durchführung benötigt wird, ist die Stückzahl, welche benötigt wird, sehr gering. Kostenersparnisse im Einkauf lassen sich nur schwer erzielen.

Zur Kontaktierung des Massestiftes mit dem Flansch wird ein separater Fügeprozess benötigt. Für konventionelle bi- und quadpolare Durchführungen handelt es sich um einen separaten Prozess. Dies kann zum Beispiel mittels eines Schweißprozesses realisiert werden, bei dem der Massestift mittels Widerstands- oder Laserschweißen elektrisch leitend an das Gehäuse angebunden wird. Dieser Prozessschritt erfordert eigene Dokumentation, Validierung bzw. Verifizierung und muss Qualitätsüberwacht werden.

Alternativ wird die Masseverbindung integriert mit den anderen Kontaktelementen mittels Hartlöten gefertigt. Es besteht hierbei jedoch die Gefahr, dass die Fügeverbindung nicht optimal auf die Masseverbindung ausgelegt ist. Bei der optischen Inspektion der Durchführungen können nur die Ober- und Unterseite des Lotes kontrolliert werden. Bei normalen Verbindungsstellen genügt zwar eine zweiseitige Kontrolle, um Aussagen darüber zu treffen, ob das Lot ausreichend aufgeschmolzen und verteilt ist. Bei Masseverbindungen kann diese Aussage aber nicht getroffen werden, da nur eine einseitige Inspektion möglich ist.

Um Aussagen über die Zugfähigkeit oder Belastbarkeit einer gefügten Masseverbindung treffen zu können, sind häufig zusätzliche Prüfungen notwendig. So können Schliffbilder oder Zugtest die Verbindung zuverlässig charakterisieren. Alternative zerstörungsfreie Verfahren (z.B. Computertomographie) sind sehr teuer und spielen in der Praxis kaum ein Rolle.

Das Herstellen und Prüfen einer separaten Fügeverbindung zur Massekontaktierierung benötigt zusätzliche Arbeitszeit. Durch das Bestücken der zusätzlichen Komponenten und das Prüfen bzw. Feststellen der Qualität der Fügeverbindung entstehen so zusätzliche Kosten, welche die Durchführung verteuern. Da der Massestift sich von den Stiften unterscheidet, unterbricht er bei vielen Arbeits- bzw. Prüfungsabläufen das normale Vorgehen und erfordert eine gesonderte Behandlung. Die gesonderten Arbeitsschritte für den Massestift müssen in der Dokumentation klar beschrieben werden. Der Massestift muss stets als solcher gekennzeichnet bzw. beschrieben werden.

Die erhöhten Erfordernisse an die Ausfallsicherheit des Massestiftes erfordern besondere Tests beziehungsweise eine spezielle Auslegung. Durch Abreißen der Masseverbindung ist die normale Signalübertragung gestört, aber das Gerät fällt nicht vollständig aus. Das Floaten der Signale kann zu nicht reproduzierbaren Systemfehlern führen und von außen nicht bzw. nur sehr schwer diagnostiziert werden.

In der WO 2013/122947 A2 wird eine Durchführung für ein implantierbares medizinisches Gerät beschrieben, bei der ein gegossener Durchführungs-Flansch eine angegossene, in der Durchführungs- Ebene liegende Lasche zur Halterung eines Massestiftes aufweist.

Der Erfindung liegt die Aufgabe zugrunde, eine verbesserte Durchführung eines implantierbaren elektromedizinischen Gerätes bereitzustellen, bei der insbesondere das erforderliche Masse-Verbindungsmittel in flexibler, kostengünstiger, langlebiger Weise realisiert ist. Insbesondere soll die Masseanbindung der Durchführung an die Elektronik die mittlere Nutzungsdauer des Implantates übersteigen und gleichzeitig der Aufwand zur Herstellung minimiert werden. Des Weiteren soll ein geeignetes Verfahren zur Herstellung einer solchen Durchführung sowie ein verbessertes implantierbares medizinelektronisches Gerät angegeben werden.

Diese Aufgabe wird in einem ersten Vorrichtungsaspekt durch eine Durchführung mit den Merkmalen des Anspruchs 1 und gemäß einem zweiten Vorrichtungsaspekt durch ein Gerät mit den Merkmalen des Anspruchs 12 sowie in ihrem Verfahrensaspekt durch ein Verfahren mit den Merkmalen des Anspruchs 9 gelöst. Zweckmäßige Fortbildungen des Erfindungsgedankens sind Gegenstand der jeweiligen abhängigen Ansprüche.

Zur Erfindung gehört der Gedanke, anstelle eines zusätzlichen Bauteils einen Abschnitt des Durchführungs-Flansches unmittelbar als Masse-Verbindungsmittel zu nutzen. Des Weiteren gehört zur Erfindung der Aspekt, dass der Durchführungs-Flansch mindestens ein vorgestanztes und gebogenes und/oder abgekantetes und/oder tiefgezogenes Blechteil, insbesondere aus einem Titanblech oder Titan-Legierungs-Blech, aufweist. Schließlich wird, in Verknüpfung der beiden vorgenannten Aspekte, vorgeschlagen, dass das Blechteil einen einstückig angeformten Fortsatz aufweist, der als Masseanschlussfläche ausgebildet ist.

In einer ersten Ausführung wird die Erfindung derart angewandt, dass die angeformte Masseanschlussfläche das alleinige Masse-Verbindungsmittel der Durchführung darstellt. Alternativ hierzu kann zusätzlich zur Masseanschlussfläche und in stoffschlüssiger Verbindung zu dieser ein Masseanschlussstift vorgesehen sein. In Abhängigkeit vom konkreten Typ des implantierbaren Geräts und der konstruktiven Ausgestaltung seiner Durchführung insgesamt kann eine der beiden Varianten bevorzugt sein. Die Anbindung der Elektronik erfolgt hierbei in zweckmäßigen Ausgestaltungen mittels Laser- oder Widerstandschweißen. Hierzu wird eine Hybrid-Leiterplatte bestehend aus einem starren und einem flexiblen Schaltungsträger hergestellt. Die Kontaktflächen des flexiblen Schaltungsträgers können elektrisch an den einzelnen Stiften bzw. dem Massestift kontaktiert und mit diesen gefügt werden.

In weiteren Ausführungen ist dem Fortsatz eine mechanisch versteifende Profil-Kontur eingeformt, etwa ein V-, U- oder Z-Profil oder eine Röhrenform. Durch das Umformen erhöht sich das Widerstandsmoment und verhindert eine ungewollte Verformung oder Deformation. Die konkrete Formgebung der versteifenden Kontur(en) kann der Fachmann aus an sich bekannten Designs zur Versteifung von Blechteilen wählen, wobei auch andere als die erwähnten Standard-Konturen zur Anwendung kommen können. Die Anbindung erfolgt ebenfalls an eine Hybrid-Leiterplatte nach Stand der Technik mittels Schweißen.

In einer weiteren Ausführung ist das freie Ende des Fortsatzes im Wesentlichen zylindrisch und mit gegenüber seinem Längsverlauf vergrößerter Fläche ausgeformt. So ist es möglich das anzuschließende Kontaktelement mit der vergrößerten Fläche des Fortsatzes besser zu kontaktieren. Hierdurch wird insbesondere eine hinreichend große Kontaktfläche zur Geräteelektronik bereitgestellt. Weiterhin wird das Anschweißen des Massestiftes erleichtert, da der der Fortsatz ausreichend Material zum Aufschmelzen und Fügen mitbringt und das Gegenelement auch während des Fügens zuverlässig fixiert und positioniert.

Um die Fügbarkeit zusätzlich zu verbessern ist es von Vorteil, wenn die Endfläche mindestens eine kleine Bohrung ∅ 0,1 mm zur Entlüftung enthält. Die Anbindung erfolgt mittels Schweißen eines auf der Platine befindlichen Stiftes. Der Gegenstift wird in die zur Hülse geformten Buchse gesteckt und elektrisch leitend verschweißt.

In einer weiteren Ausführung ist das freie Ende des Fortsatzes im Wesentlichen plan und mit gegenüber seinem Längsverlauf vergrößerter Breite ausgeformt.

Hierdurch wird insbesondere eine hinreichend große Anbindungsfläche des Flansch-Fortsatzes mit einer Anschlussfläche einer Leiterplatte (PCB) oder einer anderen Kontaktfläche zur Geräteelektronik bereitgestellt. Vorteilhaft kann es sein, diese Fläche entsprechend der Form des Landpatterns der Leiterplatte rund, sechseckig oder rechteckig auszuführen und die Größe der Flächen aufeinander abzustimmen.

Im Hinblick auf die übliche Lage der entsprechenden Leiterplatte beispielsweise im Gehäuse eines Herzschrittmachers oder implantierbaren Defibrillators kann das verbreiterte und plane Ende des Fortsatzes gegenüber dessen Längserstreckung annähernd rechtwinklig abgekantet oder entsprechend umgebogen sein, damit sich die Kontaktflächen möglichst gegenüberstehen bzw. großflächig überlappen. Zum Kontaktieren werden die beiden Flächen miteinander gefügt. Dies kann zum Beispiel mittels Schweißen, Weichlöten oder Hartlöten geschehen.

Vorteilhaft für Weichlötverbindungen können auch weitere bekannte Formen des Endabschnittes des Fortsatzes sein, zum Beispiel eine Umformung zu einem Gullwing, J-Lead oder SOP-ähnlicher Stift-Form. Vorteilhaft für Schweißverbindungen sind Einkerbungen oder Löcher zur Entlüftung des Schweißspaltes.

Zusätzlich oder auch unabhängig hiervon kann vorgesehen sein, dass das freie Ende des Fortsatzes und/oder ein Abschnitt in dessen Längsverlauf mit einem zusätzlichen (weichlötbaren) Kontaktwerkstoff versehen ist. Als Materialien für ein solchen Kontaktwerkstoff können insbesondere vorgesehen sein: Cu, Ag, Au, Ni, Pd, Pt, Ir, Fe oder diese enthaltende Legierungen, insbesondere CuAg0.10, CuAg0.10P, CuTeP. Grundsätzlich können alle bekannten Verfahren zum Aufbringen der Kontaktwerkstoffe wie Plattieren, Walzen, Schweißen, Hartlöten, Sputtern, Galvanik oder Rollnahtschweißen verwendet werden. Insbesondere die Herstellung mittels Vertikal-Drahtaufschweißen, Widerstandsschweißen und Horizontal-, Draht und Profilabschnittschweißen sind besonders gut geeignet, um lokal begrenzt auf Bänder oder Bleche im automatisierten Verfahren dünne Kontaktwerkstoffe aufzutragen.

Der Auftrag der Kontaktwarzen beträgt typischerweise einige wenige bis zu 100 µm, der typische Durchmesser kann ebenfalls im Bereich von 0,5 bis 10mm variieren und kann durch Prägen oder Taumeln in die gewünschte Form gebracht werden.

Vorteilhaft für die Oberflächenmontage (surface-mounting technology (SMT)) sind weichlötbare Kontaktwerkstoffe speziell an Teilflächen des Fortsatzes bzw. über den gesamten Bereich der vergrößerten Fläche des Fortsatzes.

Die Anbindung der Durchführung kann durch den weichlötbaren Kontaktwerkstoff auf dem Fortsatz mittels eines SMT-Prozesses direkt während der Leiterplattenbestückung erfolgen. So kann die Durchführung bereits mit anderen elektronischen Komponenten im Bestückungsautomaten auf die Leiterplatte platziert werden. Die Kontaktwarze aus einem gut weichlötbaren Material wird dabei unmittelbar in die Lötpaste auf der Platine (PCB) gesetzt. Beim Weichverlöten im Reflowverfahren wird die Lötpaste mit der Kontaktwarze gefügt und bildet einen elektrischen Kontakt. Nach dem Verlöten kann die Platine inklusive Durchführung als Ganzes geprüft werden, insbesondere können standardisierte elektrische und visuelle Testsysteme verwendet werden, z.B. automatische optische Inspektionen (AOI), Flying-Probe-Test (FPT), In-Circuit-Test (ICT), oder ähnliche.

In einer weiteren Ausführung weist der Fortsatz mindestens zwei Biegestellen im Längsverlauf zur Verleihung von Druck- und Zugelastizität und/oder zur Bereitstellung einer Längenausgleichsreserve auf. Zusätzliche Biegestellen des Fortsatzes können als federnde Elemente dienen und so Kräfte aufnehmen und kompensieren, etwa während der Gerätemontage. Eine mechanische Belastung der Fügestelle zwischen Fortsatz und Platine kann so minimiert werden. Weiterhin kann sichergestellt werden, dass die Masse-Verbindung bei mechanischer Belastung als letzter Stift abreißt, indem die Biegestellen als Ausgleichselement fungieren.

Das Ausgleichselement kann Längenänderungen oder einen axialen Versatz aufnehmen und ausgleichen. Hierzu wird die Längenausgleichsreserve des Massenfortsatzes so gewählt, dass die mittlere Längenausdehnung etwas oberhalb der normalen Stifte liegt, typischerweise 0,05 mm. So wird gewährleistet, dass der Fortsatz im leicht gestauchten Zustand zuverlässig Leiterplatte und Durchführung kontaktiert. Werden die Durchführung oder einzelne Kontakte auf Zug oder Druck belastet, bleibt sichergestellt, dass die Durchführung nicht versagt. Es ist möglich, Längenänderungen im Bereich bis zu 0,2 mm auszugleichen, und so sicherzustellen, dass die Masseverbindung als letzte von allen Signalverbindungen unterbrochen wird.

Da bei weichlötbaren Durchführungen erhöhte Anforderungen an die Koplanarität über alle Stirnflächen der Stiftenden bzw. Kontaktflächen hinweg besitzt, ist es besonders günstig, wenn der Fortsatz eine Längenausgleichsreserve zur Verfügung stellen kann. So wird verhindert, dass der Massestift für Koplanaritätsverletztungen ursächlich ist und die Wahrscheinlichkeit einer Koplanaritätsverletztung sinkt.

Unter Verfahrensaspekten gehört zur Erfindung der Gedanke, das den Fortsatz aufweisende Blechteil in einem ersten Schritt als Blech-Rohling mit einer Solltrennlinie vorzuformen und in einem hiervon zeitlich getrennten zweiten Schritt, insbesondere unmittelbar vor der Montage der Durchführung, entlang der Solltrennlinie aus dem Blech-Rohling herauszutrennen und den Fortsatz durch Umformen in Position zu bringen.

Dies ist insbesondere dann vorteilhaft, wenn die Herstellung des Durchführungs-Flansches und die Montage der Durchführung an verschiedenen Produktionsstätten stattfinden. Dann kann das überschüssige Material bei der Montage der Durchführung leicht herausgebrochen bzw. abgetrennt werden, und schützt zuvor die Masseanschlussfläche wie auch den Flansch während des Transports und vorgelagerten Prozessen vor einem Verkeilen bzw. Verhaken der Teile. Der dünne Massestift kann so vor Beschädigung und Deformation geschützt werden. Die geforderte Maßhaltigkeit kann so auch bei langen Transporten oder Vorbehandlungsprozessen ("Reinigen") als Schüttgut gewährleistet werden. In das überschüssige Material können Nuten bzw. Trennkanten zur Ausrichtung bzw. Orientierung auf der weiterverarbeitenden Maschine eingefügt werden. Eine automatisierte Weiterverarbeitung der Teile ist somit sehr einfach möglich.

Die Effizienz der Herstellung von Flanschen mit integriertem Massestift kann weiter gesteigert werden, indem zur Herstellung Bänder oder Rollen genutzt werden. Das Heraustrennen der Flansche erfolgt vollständig bis zu den Verbindungsstegen. Die Verbindungsstege schützen die Flansche und den Massestift während des Transportes vor Deformation, und gewährleisten eine gleichmäßige Zuführung in eine weiterverarbeitende Anlage. Nach dem Zuführen in eine Anlage oder dem Weiterverarbeiten kann der Verbindungssteg vom Flansch abgetrennt werden. Hierfür kann in einem Ausführungsbeispiel in einem Endabschnitt des Verbindungsstegs eine Sollbruchstelle eingebracht werden. Dies kann durch Einbringen einer Einkerbung, mindestens einer durchgehender Ausnehmung in diesem Bereich, oder einer gezielten Verjüngung des Materials realisiert werden. Das Zuführen der gestanzten Teile von Rollen oder Bändern ist besonders wirtschaftlich für mittlere und große Stückzahlen.

In einer Ausgestaltung des Verfahrens ist vorgesehen, dass mindestens der Fortsatz des Blechteils oder ein als Ausgangsmaterial dienendes Blech mindestens bereichsweise mit einer polymeren oder anderen organischen Schutzschicht als Oxidationsschicht versehen wird (OSP- Organic Surface Protection). Bekannte Schutzfilme (z.B. Glicoat) können vollständig oder selektiv auf den Stift nach dem teilweisen oder vollständigen Heraustrennen abgeschieden werden. Typische Schichtdicken betragen 0,2 bis 0,6 µm und bestehen zum Beispiel aus substituierten Imidiazolen und/oder Triazolen. Die Schutzfilme verhindern die Oxidation des Grundmaterials während der Lagerung typischerweise für mehrere Monate und pyrolysieren unmittelbar vor bzw. während des Hartlöt- bzw. Weichlötprozesses.

Das vorgeschlagene implantierbare Gerät, welches eine Durchführung mit wenigstens einem der oben beschriebenen Aspekte umfasst, ist insbesondere als Herzschrittmacher oder implantierbarer Kardioverter oder Cochlearimplantat ausgebildet. Es können aber auch andersartige implantierbare medizinische Geräte mit der erfindungsgemäßen Durchführung ausgestattet werden, sofern diese ein Masse-Verbindungsmittel benötigen und bei ihnen ein mindestens gebogenes und/oder abgekantetes und/oder tiefgezogenes Blechteil als Bestandteil des Durchführungs-Flansches einsetzbar ist.

Einerseits kann bei einem solchen Gerät allein der angeformte Fortsatz als Masse-Verbindungsmittel des elektrischen oder elektronischen Bauelements vorgesehen und hierzu direkt stoffschlüssig mit dem Bauelement, insbesondere einem Leitungsträger, verbunden sein. Alternativ hierzu können der Fortsatz und ein zusätzlicher Masseanschlussstift, welcher einerseits stoffschlüssig mit dem Bauelement, insbesondere einer Leiterplatte oder einen anderen Leitungsträger, und andererseits mit dem Fortsatz stoffschlüssig verbunden ist, gemeinsam als Masse-Verbindungsmittel des Bauelements vorgesehen sein. Obgleich sich bei einer solchen Kombinations-Lösung nicht alle der grundsätzlich erzielbaren Vorteile der Erfindung realisieren lassen, kann diese speziell unter dem Aspekt der Zuverlässigkeit der Masseverbindung, aber auch hinsichtlich gewisser technologischer Vereinfachungen, einen Gewinn gegenüber bekannten Konfigurationen erbringen.

Mit der Erfindung lassen sich, zumindest in bestimmten Ausführungen (wie weiter oben beispielhaft erläutert), insbesondere ein oder mehrere der folgenden Vorteile erreichen:
- Da keine Übergänge oder Materialkombinationen am Übergang zwischen Flansch und Masseverbindung entstehen, ist die Wärmeausdehnung gleichartig, so dass thermische oder mechanische Beanspruchung das Bauteil nicht belastet.
- Die Realisierung der Masseverbindung ist technologisch höchst einfach und somit kostengünstig möglich, auch wegen des Fortfalls von Prüfschritten und entsprechenden Dokumentationen. Durch das Stanzen lassen sich die Flansche mit integrierter Masseanschlussfläche in hoher Stückzahl sehr kostengünstig produzieren. Da sich stets mehrere Schneiden im Eingriff befinden, können viele Flansche parallel gefertigt werden, so dass der Durchsatz der Produktion steigt. Die Montagezeit der Durchführungen verringert sich um die üblicherweise für die Schritte des Zuführens und Einbindens von Stift, Lot und Lötpad benötigten Zeitspannen. Durch die Integration von weichlötbaren Materialien auf dem Kopf des Stiftes bzw. in das Halbzeug des Bleches kann zusätzlich Bestückungszeit eingespart werden.
- Es gibt nennenswerte Vereinfachungen in der Logistik der zur Herstellung eines implantierbaren Gerätes benötigten Teile, einschließlich der zugehörigen Einkaufs- und Lagerhaltungs-Software.
- Es können Verbesserungen bei der Zuverlässigkeit des Gerätes erwartet werden, da die Zahl möglicher Fehlerquellen gegenüber Lösungen, die zusätzliche Teile benötigen, deutlich reduziert ist. Durch den Fortfall einer Fügestelle zwischen Masseverbindung und Flansch können hier auch keine Produktionsfehler, Diffusionsfehler oder Korrosionsprobleme auftreten. Die Durchgängigkeit des Materials von Flansch zu Anschlussfläche schließt thermoelektrische Effekte an den Übergangsstellen aus. Gleichspannungsoffsets können somit systematisch ausgeschlossen werden. Da keine Übergänge oder Materialkombinationen am Übergang zwischen Flansch und Masseanschlussfläche entstehen, ist die Wärmeausdehnung gleichartig, so dass thermische oder mechanische Beanspruchungen das Bauteil nicht belasten.
- Durch den Wegfall der Fügeverbindung mit mindestens zwei bis drei unterschiedlichen Materialen entfallen die typischen Gefüge- und Strukturänderungen, intermetallischen Phasen sowie Kornbereichsgrenzen.
- Durch den Einsatz einer Längenausgleichsreserve kann die Koplanarität der Durchführung verbessert werden, und der Ausfall des Implantates bei extremen Vibrations- und Schockbelastungen kann minimiert werden.

Vorteile und Zweckmäßigkeiten der Erfindung ergeben sich im Übrigen aus der Beschreibung von Ausführungsbeispielen anhand der Figuren. Von diesen zeigen:
- Fig. 1: eine schematische, teilweise geschnittene Darstellung eines implantierbaren elektromedizinischen Geräts,
- Fig. 2: eine perspektivische Darstellung eines Blechteils zur Herstellung eines Durchführungs-Flansches gemäß einer Ausführungsform der Erfindung,
- Fig. 3A bis 3C: schematische Längsschnittdarstellungen von Durchführungen gemäß weiteren Ausführungsbeispielen der Erfindung,
- Fig. 4: eine schematische Draufsicht auf ein Blechteil eines Durchführungsflansches gemäß einem weiteren Ausführungsbeispiel und
- Fig. 5A und 5B: schematische perspektivische Darstellungen (Detailansichten) von Durchführungs-Flanschen gemäß weiteren Ausführungsbeispielen der Erfindung.
- Fig. 6: schematische perspektivische Darstellung eines fertigen Durchführung-Flansches
- Fig. 7: schematische Darstellung eines Stanzkamms

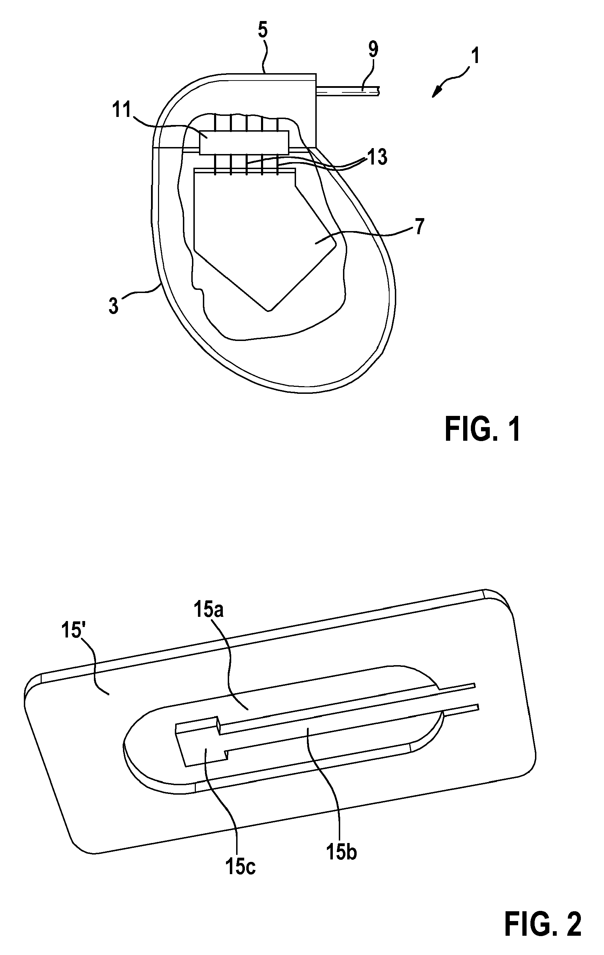

Fig. 1 zeigt einen Herzschrittmacher 1 mit einem Schrittmachergehäuse 3 und einem Kopfteil (Header) 5, in dessen Inneren neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11 umfasst eine Mehrzahl von Anschlussstiften 13. Die Anschlussstifte sind an einem Ende durch eine entsprechende Bohrung in die Leiterplatte gesteckt und mit dieser weich verlötet.

Fig. 2 zeigt in schematischer perspektivischer Darstellung ein Blechteil 15', das aus einem Blech eines zur Herstellung eines Durchführungs-Flansches geeigneten Materials ausgestanzt ist und nach Umformschritten einen einfach und kostengünstig herzustellenden Durchführungs-Flansch ergibt. Das Blechteil 15' hat eine annähernd rechteckige Außenkontur mit abgerundeten Ecken, und innen ist eine ebenfalls annähernd rechteckige Ausnehmung 15a mit gegenüberliegenden halbkreisförmigen Endbereichen ausgestanzt.

Innerhalb dieser Ausnehmung ist ein langgestreckt rechteckiger Fortsatz 15b mit verbreitertem, annähernd quadratischem Endabschnitt 15c stehen geblieben. Im Rahmen der weiteren Umformungsschritte des Blechteils 15' wird der Fortsatz 15b annähernd rechtwinklig nach unten abgekantet, und der Endabschnitt 15c kann gegenüber diesem Längsverlauf des Fortsatzes nochmals annähernd rechtwinklig abgekantet sein, so dass er letztlich wieder parallel zur Erstreckungsebene des Flansches und somit parallel zur Erstreckung einer in typischer Weise im Gehäuse des Gerätes 1 (Fig. 1) angeordneten Leiter- bzw. Anschlussfläche ausgerichtet ist. Er kann damit leicht mit jener Anschlussfläche stoffschlüssig verbunden, insbesondere verlötet, werden.

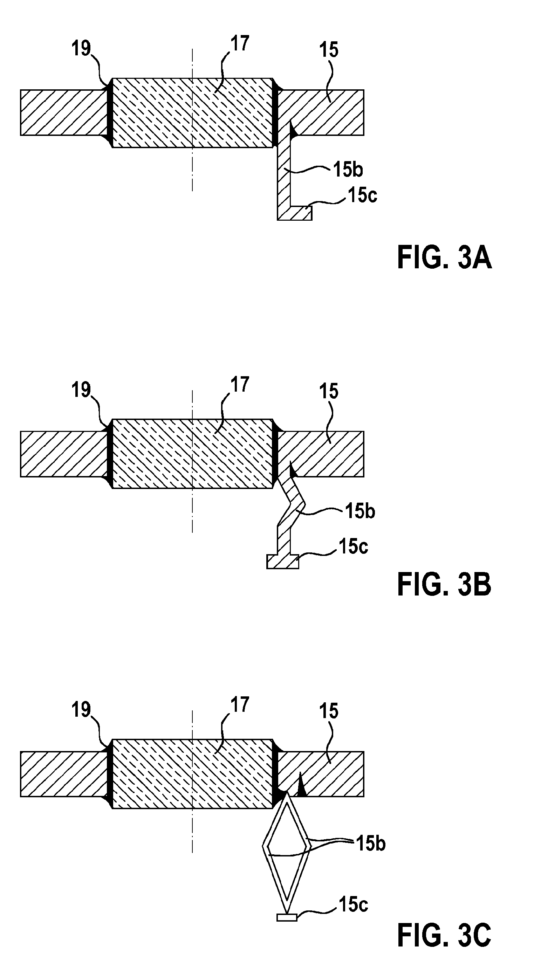

Ein solcher Zustand ist in Fig. 3A als schematische Längsschnittdarstellung gezeigt. Diese Figur zeigt neben dem Durchführungs-Flansch 15 einen in diesen eingesetzten Grund- bzw. Isolierkörper 17. In jenen sind typischerweise ein oder mehrere Anschlussstifte zum signalmäßigen externen Anschluss elektronischer/elektrischer Komponenten des Gerätes in der schematisch in Fig. 1 gezeigten Art eingebunden, solche Anschlussstifte sind aber in Fig. 3a nicht gezeigt. Eine umlaufende Hartlötverbindung 19 um die Außenkante des Grund- bzw. Isolierkörpers 17 befestigt diesen im Durchführungs-Flansch 15 und gewährleistet zugleich eine hermetische Abdichtung, die sich auch über (nicht gesondert bezeichnete) Einschnitte zwischen dem Fortsatz 15b und dem umgebenden Flanschmaterial erstreckt und somit auch dort eine Abdichtung und Versiegelung bewirkt. Dabei werden entstandene Kanten bzw. durch das Biegen entstandene Radien durch Hartlot (z.B. Gold) abgedeckt und versiegelt. Potentielle Leckpfade im Bereich des Massestiftes werden somit durch den normalen Hartlötprozess (brazing) dauerhaft geschlossen. Während der normalen Lecksuche können Undichtheiten bis zu einem Grenzwert von 1e-11mbar l/s detektiert werden.

Fig. 3B und 3C zeigen Modifikationen in der Formgebung des die Masseanschlussfläche des implantierbaren Gerätes bildenden Fortsatzes, wobei trotz unterschiedlicher Ausgestaltung die gleichen Bezugsziffern wie in Fig. 2 und 3A benutzt werden. Bei der in Fig. 3B gezeigten Ausführung sind im Längsverlauf des Fortsatzes 15b drei Knickstellen vorgesehen, die eine Federwirkung bzw. ein elastisches Nachgeben der Masseverbindung und somit eine erhöhte mechanische Belastbarkeit derselben gewährleistet. Vorteilhaft ist dies insbesondere im Hinblick darauf, dass in einem Gerät der in Rede stehenden Art bei großer mechanischer Belastung die Masseverbindung als letzte von allen in der Durchführung realisierten Verbindungen unterbrochen werden sollte, so dass keine sog. floatenden Signale entstehen. Auch die Ausführung gemäß Fig. 3C, bei der der Fortsatz 15b in Längsrichtung bis zum Beginn des Endabschnitts 15c aufgespalten ist und seine beiden Teile beim Umformen in gegenüberliegende Richtungen ausgespreizt wurden, lassen sich diese Vorteile geltend machen.

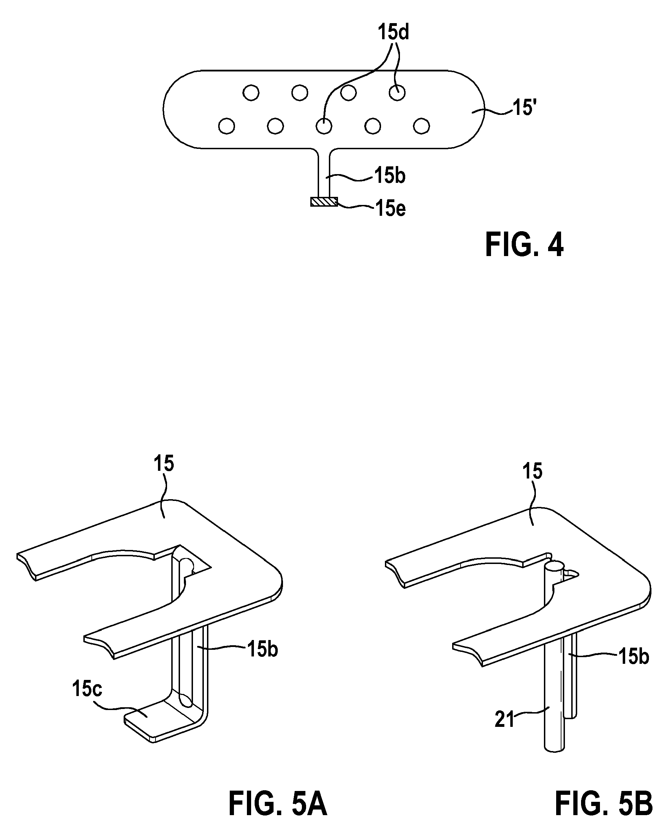

Fig. 4 zeigt, wiederum unter Verwendung der gleichen Bezugsziffern wie in den Figuren 2 bis 3c, eine weitere modifizierte Konfiguration. Hier ist das Blechteil 15'mit mehreren kleineren Ausnehmungen 15d versehen, in denen später dünne Isolationskörper Platz finden sollen. Der die Masseanschlussfläche realisierende Fortsatz 15b mit seinem verbreiterten Endabschnitt 15c ist deshalb an die Außenkontur des Flansches 15 angeformt. Der Fortsatz ist hier im noch unverformten Zustand gezeigt, aber sein Endabschnitt 15c ist bereits mit einer weichlötbaren Beschichtung 15e zur Erleichterung des späteren Schrittes eines Verbindens mit Leiter- bzw. Anschlussflächen im Gerät versehen.

Generell ist zu den Herstellungs- bzw. Verarbeitungsschritten von Blechteilen für Durchführungs-Flansche der in Fig. 1 und 4 gezeigten Art folgendes anzumerken:
Findet die Herstellung des Flansches und die Montage der Durchführung an verschiedenen Produktionsstätten statt, so kann es von Vorteil sein, den Fortsatz (die Anschlussfläche) und den Flansch mit einer Sollbruchstelle bzw. Perforation und überschüssigem Material zu fertigen. So kann das überschüssige Material bei der Montage leicht herausgebrochen bzw. abgetrennt werden. Es schützt den Fortsatz und den Flansch während des Transports und vorgelagerten Prozessen noch vor einem Verkeilen bzw. Verhaken der Teile. Die geforderte Maßhaltigkeit der Teile kann so auch für den Transport als Schüttgut gewährleistet werden. In das überschüssige Material können Nuten bzw. Trennkanten zur Ausrichtung bzw. Orientierung auf der weiterverarbeitenden Maschine eingefügt werden. Eine automatisierte Weiterverarbeitung der Teile ist somit sehr einfach möglich.

Um die Fügbarkeit des Stiftes an der Platine zu erhöhen, können auf dem Stift bereits auf Halbzeugebene Kontaktstellen aufgebracht werden. Diese können zum Beispiel mittels Schweißen oder Hartlöten gefügt werden. Es ist vorteilhaft, vor dem Heraustrennen im Bereich des Stiftkopfes bzw. Pads auf dem Halbzeug des Masterbleches eine Fläche aus Nickel oder Kupfer bzw. einem anderen gut weichlötbaren Material zu fügen. Die Fügestelle muss dabei keine erhöhten Anforderungen an Maßhaltigkeit erfüllen, da im Anschließenden Prozessschritt mittels Trennen der umliegende Bereich des Materials herausgetrennt wird.

Vorteilhaft kann es sein, Flächen des Fortsatzes oder auch des ganzen Blechteils oder dessen Beschichtung bis zur weiteren Verarbeitung mit einem Polymer oder einem organischen Schutzfilm (OSP - Organic Surface Protection) zu versiegeln. Bekannte Schutzfilme können vollständig oder selektiv auf den Stift nach dem teilweisen oder vollständigen Heraustrennen abgeschieden werden. Typische Schichtdicken betragen 0,2 bis 0,6µm und bestehen zum Beispiel aus substituierten Imidiazolen und/oder Triazolen. Die Schutzfilme verhindern die Oxidation des Grundmaterials während der Lagerung typischerweise für mehrere Monate und pyrolysieren unmittelbar vor bzw. während des Hartlöt- bzw. Weichlötprozesses.

Fig. 5A zeigt skizzenhaft eine weitere Modifikation, bei der der als Masseanschlussfläche des Gerätes dienende Fortsatz 15b in seinem Querschnitt V-förmig konturiert ist, um ihn zu versteifen. Der Endabschnitt 15c ist jedoch auch hier eben ausgeführt, um eine brauchbare Lötfläche zu bilden. Eine spezielle Verbreiterung gegenüber dem übrigen Längsverlauf des Fortsatzes ist bei dieser Ausführung allerdings nicht vorgesehen.

Fig. 5B zeigt eine ähnliche Ausgestaltung, allerdings statt V-förmiger mit halbkreisförmiger Konturierung des Fortsatzes 15b und ohne abgekanteten bzw. umgebogenen Endabschnitt. Dieser wird hier nicht benötigt, denn bei dieser Konfiguration ist als zusätzliches Masseverbindungs-Element ein mit dem Fortsatz 15b verlöteter Massepin 21 vorgesehen. Es ist letztlich dessen Ende, welches auf eine korrespondierende Anschlussfläche an einem Leitungsträger des Gerätes aufgelötet wird. Es wird darauf hingewiesen, dass diese Ausführung wegen ihrer technologischen und kostenseitigen Nachteile aus derzeitiger Sicht nur für spezielle Anwendungen der vorliegenden Erfindung in Betracht zu ziehen ist. Ein spezieller Vorteil dieser Kombination ist die Schaffung einer großflächigen und außerordentlich zuverlässigen mechanischen und elektrischen Verbindung zwischen dem Masseverbindungselement und dem eigentlichen Durchführungs-Flansch.

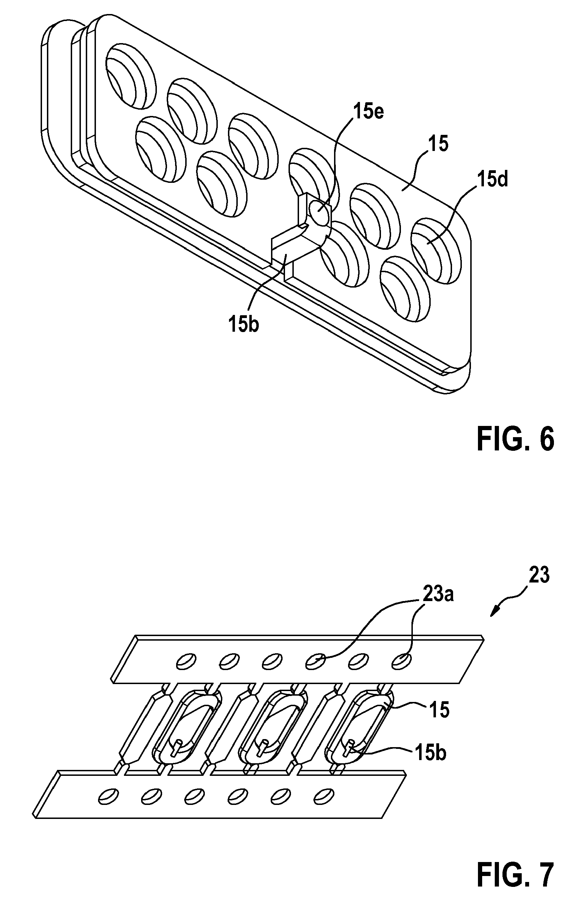

Fig. 6 zeigt den fertigen Flansch 15 einer zehnpoligen ICD Durchführung mit gestanztem Massestift 15b. In jede der Ausnehmungen 15d des Flansches kann eine Isolationskeramik sowie ein Stift zur Signalleitung gefügt werden. Auf dem Massestift befindet sich eine Kontaktstelle 15e aus gut weichlötbarem Material, hier NiAg, welche vor dem Stanzen der Flanschkontur mittels Schweißen auf den Grundwerkstoff gefügt wurde. Der Massestift 15b wurde nach dem Stanzen des Flansches mehrmals umgeformt, dabei wird der Fortsatz mit der Kontaktstelle 15e aufgestellt und an seine Position gebogen. Nach dem Fügen von Keramik, Stiften und Flansch schließen die Stifte und die Kotaktwarze auf der Ausnehmung koplanar, d.h. in einer gemeinsamen Ebene ab. Die erlaubte Abweichung beträgt typischerweise 0,1mm.

Fig. 7 zeigt exemplarisch einen Stanzkamm 23 nach Produktion für ICD Flansche mit Massestiften 15b. Das ursprüngliche Werkstoffband wird mittels der Löcher 23a im Werkzeug orientiert, geführt und synchronisiert. Schrittweise werden die Konturen herausgestanzt, abgekantet und ggf. umgeformt. Nach der Herstellung können die Flansche 15 an den Bruchkanten manuell oder automatisiert aus dem Stanzkamm 23 entnommen werden. Das Umformen bzw. Aufstellen des Massestiftes 15b kann in einer zweiten Anlage oder ggf. integriert im Prozess erfolgen.

Die Ausführung der Erfindung ist auch in einer Vielzahl von Abwandlungen der hier gezeigten Beispiele und weiter oben hervorgehobenen Aspekte der Erfindung möglich.

## Patentansprüche

1. Durchführung (11) eines implantierbaren medizinelektronischen Geräts (1), mit einem Isolierkörper (17), einem den Isolierkörper umfassenden Durchführungs-Flansch (15) und mindestens einem den Isolierkörper durchstoßenden Anschlusselement (13, 15b) zum externen Anschluss eines elektrischen oder elektronischen Bauelements (7) des Geräts,
wobei der Durchführungs-Flansch mindestens ein vorgestanztes und gebogenes und/oder abgekantetes und/oder tiefgezogenes Blechteil, insbesondere aus einem Titanblech oder Titan-Legierungs-Blech, aufweist und
das Blechteil einen einstückig angeformten und gegenüber der Erstreckungsebene der Durchführung abgewinkelten Fortsatz (15b) aufweist, der als Masseanschlussfläche ausgebildet ist.

2. Durchführung nach Anspruch 1, wobei die angeformte Masseanschlussfläche (15b) das alleinige Masse-Verbindungsmittel der Durchführung darstellt.

3. Durchführung nach Anspruch 1, in der zusätzlich zur Masseanschlussfläche (15b) und in stoffschlüssiger Verbindung zu dieser ein Masseanschlussstift (21) vorgesehen ist.

4. Durchführung nach einem der vorangehenden Ansprüche, wobei dem Fortsatz (15b) eine mechanisch versteifende Profil-Kontur eingeformt ist, insbesondere ein V-, U- oder Z-Profil oder eine Röhrenform.

5. Durchführung nach einem der vorangehenden Ansprüche, wobei das freie Ende (15c) des Fortsatzes (15b) im Wesentlichen plan und mit gegenüber seinem Längsverlauf vergrößerter Fläche ausgeformt ist.

6. Durchführung nach einem der vorangehenden Ansprüche, wobei das freie Ende (15c) des Fortsatzes (15b) und/oder ein Abschnitt in dessen Längsverlauf mit einer weichlötbaren Beschichtung (15e) versehen ist.

7. Durchführung nach einem der vorangehenden Ansprüche, wobei der Fortsatz (15b) mehrere Biegestellen im Längsverlauf zur Verleihung von Druck- und Zugelastizität und/oder zur Bereitstellung einer Längenausgleichsreserve aufweist.

8. Durchführung nach einem der vorangehenden Ansprüche, wobei der Durchführungs-Flansch (15) aus mehreren vorgeformten Teilen gefügt ist und insbesondere einen mehrlagigen Blechteil-Verbund umfasst, und die Masseanschlussfläche (15b) aus einem einzelnen Teil (15') gebildet ist.

9. Verfahren zur Herstellung einer Durchführung (11) nach einem der vorangehenden Ansprüche, wobei das den Fortsatz (15b) aufweisende Blechteil (15') in einem ersten Schritt als Blech-Rohling (23) mit einer Solltrennlinie vorgeformt und in einem hiervon zeitlich getrennten zweiten Schritt, insbesondere unmittelbar vor der Montage der Durchführung, entlang der Solltrennlinie aus dem Blech-Rohling herausgetrennt wird.

10. Verfahren zur Herstellung einer Durchführung (11) nach einem der vorangehenden Ansprüche, wobei mindestens der Fortsatz (15b) des Blechteils (15') oder ein als Ausgangsmaterial dienendes Blech mindestens bereichsweise mit einer, insbesondere polymeren oder anderen organischen, Schutzschicht als Oxidationsschicht versehen wird.

11. Verfahren nach Anspruch 10, wobei die Schutzschicht als Dünnschicht abgeschieden wird.

12. Implantierbares medizinisches Gerät (1) mit einer Durchführung (11) nach einem der Ansprüche 1 bis 8.

13. Gerät nach Anspruch 12, ausgebildet als Herzschrittmacher (1) oder implantierbarer Kardioverter oder Cochlearimplantat.

14. Gerät nach Anspruch 12 oder 13, wobei allein der angeformte Fortsatz (15b) als Masse-Verbindungsmittel des elektrischen oder elektronischen Bauelements (7) vorgesehen und hierzu direkt stoffschlüssig mit dem Bauelement, insbesondere einem Leitungsträger, verbunden ist.

15. Gerät nach Anspruch 12 oder 13, wobei der Fortsatz (15b) und ein zusätzlicher Masseanschlussstift (21), welcher einerseits stoffschlüssig mit dem Bauelement (7), insbesondere einem Leitungsträger, und andererseits stoffschlüssig mit dem Fortsatz verbunden ist, gemeinsam als Masse-Verbindungsmittel des Bauelements vorgesehen sind.
